Europäisches Patentamt

European Patent Office

Office européen des brevets

(19)

(11) **EP 1 520 615 A1**

(12) ## DEMANDE DE BREVET EUROPEEN

(43) Date de publication:
**06.04.2005 Bulletin 2005/14**

(51) Int Cl.7: **B01D 53/88**, B01D 53/86,
A61L 9/20, E04C 2/00

(21) Numéro de dépôt: **03447240.7**

(22) Date de dépôt: **01.10.2003**

(84) Etats contractants désignés:
**AT BE BG CH CY CZ DE DK EE ES FI FR GB GR
HU IE IT LI LU MC NL PT RO SE SI SK TR**
Etats d'extension désignés:
**AL LT LV MK**

(71) Demandeur: **R & D du groupe Cockerill-Sambre
4000 Liège (BE)**

(72) Inventeurs:
• **Dehbi, Leila
1160 Auderghem (BE)**

• **Geron, Laurent
4317 Les Waleffes (BE)**
• **Winand, René
1330 Rixensart (BE)**

(74) Mandataire: **Van Malderen, Michel et al
Office van Malderen
85/043 Boulevard de la Sauvenière
4000 Liège (BE)**

(54) **Paroi de purification d'air**

(57)    Appareil de purification photocatalytique en continu de l'air d'une pièce d'habitation, se présentant de préférence sous forme d'une paroi murale (1), comprenant une structure externe en acier, une ouverture (11) pour l'entrée de l'air à traiter, une ossature métallique interne (4) sur laquelle est fixée une pluralité de lampes UVA (3), un filtre photocatalytique (2) et une ouverture (12) pour la sortie de l'air purifié, caractérisé en ce que le filtre photocatalytique comprend au moins une grille (2) en métal déployé recouverte d'un film comprenant du dioxyde de titane ($TiO_2$), majoritairement en phase anatase pour maximiser la surface de photocatalyseur éclairée par la lumière UVA.

FIG.2

EP 1 520 615 A1

## Description

### Objet de l'invention

La présente invention se rapporte à un appareil de purification d'air fonctionnant sur le principe de la photocatalyse hétérogène au contact du dioxyde de titane et se présentant de préférence sous la forme d'une paroi murale.
L'invention concerne également le procédé de purification d'air mis en oeuvre au moyen dudit appareil.

### Arrière-plan technologique et état de la technique

On connaît la photocatalyse hétérogène depuis les années 70 suite à des études sur la dissociation photoinduite de l'eau. Cette technique consiste à irradier par la lumière solaire naturelle ou par un éclairage artificiel UV ($\lambda <$ 400 nm) un semiconducteur, généralement le dioxyde de titane. Ce matériau subit une excitation qui permet à un électron $e^-$ de la bande de valence d'être éjecté dans la bande de conduction du semiconducteur (réduction). Le trou $h^+$ correspondant (oxydation) peut réagir avec un groupement OH adsorbé à la surface du semiconducteur pour former des radicaux hydroxyle OH très oxydants. Ceux-ci sont capables de réagir avec des molécules organiques, par exemple des polluants, pour conduire à la minéralisation de ceux-ci avec formation d'eau et de dioxyde de carbone. La photocatalyse est une accélération de la photoréaction par la présence du catalyseur. Elle est hétérogène car les photoréactions se produisent à l'interface entre deux milieux se trouvant dans des phases différentes à la surface du catalyseur.
Le dioxyde de titane $TiO_2$ existe sous différentes formes cristallines : le rutile, l'anatase, la brookite et un grand nombre de phases obtenues par haute pression. Seules les formes cristallines rutile et anatase ont une activité photocatalytique. En particulier, l'anatase, la forme la plus active, qui a été utilisée dans le cadre de la présente invention, possède une structure tétraédrique allongée avec des octaèdres d'oxygène irréguliers.
Le dioxyde de titane est présent en grande quantité, que ce soit dans les peintures, les cosmétiques, l'alimentaire, etc. Sa photoactivité permet donc de l'utiliser pour la décomposition de molécules organiques adsorbées à sa surface. La photocatalyse hétérogène au contact du dioxyde de titane a donc été utilisée notamment pour la purification de l'eau, la destruction de polluants, pesticides, colorants, bactéries, la détoxification des eaux de rinçage agricoles et industrielles, la purification de l'air (désodorisation, élimination de gaz nocifs) et comme agent autonettoyant d'objets ou d'édifices en plein air, exposés aux intempéries.
Un très grand nombre d'appareils de purification d'air et/ou désodorisation contenant un filtre photocatalytique à base de $TiO_2$, dont certains possèdent également une fonction structurelle ou architecturale, ont été proposés. La plupart du temps, les problèmes solutionnés par ces inventions sont :

- optimisation de l'utilisation du rayonnement UV, réalisée par exemple grâce à l'optimisation des géométries et arrangements entre le support photocatalytique et les lampes ou par augmentation de l'efficacité d'une lampe UV pour l'action photocatalytique par la mise en place de réflecteurs (voir par ex. JP 09 084866, EP 993859, JP 2000 334448, JP 10 249166, JP 2001 293336, JP 2002 295874, JP 2001 218820) ;
- augmentation de l'étendue du spectre de lumière utilisable pour l'activité catalytique, par exemple utilisation de la lumière visible dans des applications de type luminaire photocatalytique (voir par ex. JP 2002 083511, JP 2002 035599);
- amélioration de la maintenance des appareils, suite au remplacement facilité des lampes ;
- augmentation de la compacité par utilisation de lampes planes ou diodes électoluminescentes (LED) plutôt que les lampes UV traditionnelles (voir par ex. JP 2000 051332, JP 09 000941).

Dans la plupart des cas, l'appareil breveté est muni d'un ventilateur pour forcer la circulation d'air, le flux d'air étant éventuellement rendu turbulent.
Cependant, les appareils de l'état de la technique sont peu ou pas satisfaisants pour ce qui concerne les exigences suivantes :

- l'incorporation du purificateur d'air comme élément architectural demande d'une part des propriétés mécaniques structurales de l'ensemble qui soient satisfaisantes et d'autre part une grande compacité en profondeur, ce qui est difficile à réaliser vu la nécessité de disposer les lampes UV avec une orientation correcte ;
- maximisation de la surface du filtre photocatalytique atteinte par l'éclairage UV ;
- optimisation de la circulation d'air à l'intérieur de l'appareil ;
- contrôle de la température des parois externes de l'appareil.

**Buts de l'invention**

La présente invention vise à fournir une solution qui permette de s'affranchir des inconvénients de l'état de la technique.

L'invention a pour but de fournir un purificateur d'air intégré aux éléments de construction des bâtiments au niveau de leurs parois murales.

En particulier, l'invention a encore pour but de fournir un purificateur d'air fonctionnant en boucle ouverte de manière à dépolluer en continu l'atmosphère d'un espace habitable.

**Principaux éléments caractéristiques de l'invention**

Un premier objet de la présente invention se rapporte à un appareil de purification photocatalytique en continu de l'air d'une pièce d'habitation, se présentant de préférence sous forme d'une paroi murale, comprenant :

- une structure externe métallique, de préférence en acier ;
- une ouverture pour l'entrée de l'air à traiter située dans la partie inférieure de la face avant de la paroi ;
- une ossature métallique interne sur laquelle est fixée une pluralité de lampes UVA, c'est-à-dire émettant un rayonnement ultraviolet dans l'intervalle 315-400 nm ;
- un filtre comprenant un support recouvert d'un film comprenant du dioxyde de titane ($TiO_2$) photocatalytique ;
- une ouverture pour la sortie de l'air purifié située dans la partie supérieure de la face avant de la paroi, l'écoulement de l'air dans l'appareil étant assurée par circulation naturelle ou forcée par au moins un ventilateur ;

caractérisé en ce que ledit appareil comprend au moins une grille en métal déployé recouverte d'un film comprenant du dioxyde de titane ($TiO_2$), majoritairement en phase anatase, pour maximiser la surface de photocatalyseur éclairée par la lumière UVA.

La présente invention présente l'avantage sur l'état de la technique que le support du filtre photocatalytique est en métal et possède donc une durée de vie illimitée, contrairement aux supports en papier habituellement utilisés.

L'appareil de purification d'air ambiant de l'invention est préférentiellement destinée à la destruction par photocatalyse au contact du dioxyde de titane de composés volatiles organiques tels que alcanes, alcools, aldéhydes, cétones, aromatiques et terpènes.

Cet appareil est destiné plus généralement à une utilisation dans le secteur du bâtiment, sous la forme d'un élément de structure ou décoratif tel que par exemple une paroi murale, une cloison, un plancher, un plafond, un faux-plafond, etc. L'élément de structure présentant une face externe métallique est compatible avantageusement avec tout type de finition de recouvrement tel que plâtre, peinture, papier peint, etc.

Avantageusement, la structure externe métallique est soit réalisée dans un acier tel qu'un acier inoxydable recuit brillant, soit encore possède une surface interne recouverte d'une couche mince, présentant un facteur de réflexion supérieur à 90% pour les longueurs d'onde inférieures à 400 nm.

Le fait de maximiser d'une part la surface totale éclairée et d'autre part la puissance reçue par unité de surface du filtre présente l'avantage de pouvoir réduire sensiblement la puissance d'éclairage nécessaire, et partant l'échauffement et le coût de fonctionnement, et en fin de compte d'optimiser le rendement.

Selon une modalité préférée de l'invention, la face avant de la structure externe a une largeur d'au moins 1,5 mètre, de préférence 2 mètres, les ouvertures d'entrée et de sortie d'air ayant la forme de fentes de largeurs égales et de valeur légèrement inférieure à celle de la largeur de ladite face avant et de hauteurs supérieures à 3 cm, de préférence égales à 5 cm.

De préférence, lesdites ouvertures sont situées à moins de 10 cm, de préférence encore 5 cm, des extrémités respectivement basse et haute de ladite face avant.

Une caractéristique essentielle de l'invention étant de maximiser la surface de métal déployé, de préférence un acier déployé, recouverte de photocatalyseur, toute la surface des mailles de cet acier déployé ($S_{acier}$) est recouverte du film de $TiO_2$, à l'exclusion de la surface de l'épaisseur de la maille, c'est-à-dire :

$$S_{acier} = [av_{maille} \sqrt{(\frac{DL_{maille}}{2})^2 + (\frac{DC_{maille}}{2})^2} - \frac{av_{maille}^2}{2\sin(2\,arctg(\frac{DC_{maille}}{DL_{maille}}))}]$$

où $DL_{maille}$, $DC_{maille}$ et $av_{maille}$ sont respectivement la diagonale longue, la diagonale courte et la lanière de la maille.

En vue de renforcer encore cet avantage, la maille est choisie pour minimiser le rapport ($S_{acier}$) entre sa surface physique ($S_{acier}$) et sa surface totale ($S_{maille}$) :

$$S_{acier} = \frac{S_{acier}}{S_{maille}} = \frac{4}{DL_{maille}DC_{maille}}\ S_{acier},$$

ledit rapport valant de préférence 1/3.

Selon des formes d'exécution préférées de l'invention, la grille est maintenue verticalement avec des fixations situées uniquement sur le pourtour de la grille, les lampes UVA sont disposées par séries de trois sur la largeur de la paroi, le ventilateur est de type tangentiel à 90° et est situé en haut de la paroi, le nombre de ventilateurs étant choisi pour obtenir un renouvellement d'air d'au moins 30 m$^3$/heure/personne dans la pièce d'habitation.

Selon une autre modalité encore préférée de l'invention, l'appareil purificateur d'air peut prendre la forme d'un cylindre de section circulaire, rectangulaire ou carrée présentant au moins un tube d'éclairage UVA selon l'axe du cylindre et entouré d'une grille en métal déployé recouverte de TiO$_2$ photocatalytique, la surface interne du cylindre ayant un facteur de réflexion supérieur à 90%. Cette modalité d'exécution est plus particulièrement destinée à la purification ou la dépollution de l'air dans un conduit.

Un deuxième objet de la présente invention se rapporte à un procédé d'optimisation du dimensionnement d'un élément purificateur d'air tel que détaillé ci-dessus, ce procédé étant caractérisé par les étapes suivantes :

a) définition de la géométrie externe de l'appareil ;

b) définition du nombre de grilles en acier déployé et du dispositif d'éclairage ;

c) calcul de l'éclairement des grilles ; si l'intensité lumineuse n'est pas supérieure au seuil de tolérance fixé, retour à l'étape b) ;

d) calcul de l'écoulement de l'air et de la distribution de température ; s'il y a échauffement des parois en acier, retour à l'étape b) ;

e) calcul de l'évolution de la concentration des polluants ; si le rendement global de purification n'est pas supérieur à la limite prédéfinie, retour à l'étape b) ;

f) obtention de l'élément de purification ou dépollution de dimensions optimales.

## Brève description des figures

La figure 1 montre schématiquement une vue tridimensionnelle de la paroi purificatrice d'air selon la présente invention.

La figure 2 représente schématiquement une vue éclatée de la paroi active selon l'invention.

La figure 3 représente schématiquement une maille, unité fondamentale d'un élément en métal déployé, sur lequel le dioxyde de titane est déposé.

La figure 4 représente schématiquement la modification d'écoulement dans un ventilateur tangentiel à 90°.

La figure 5.a représente la carte d'éclairement brute calculée sur une grille de métal déployé appartenant au purificateur d'air selon l'invention.

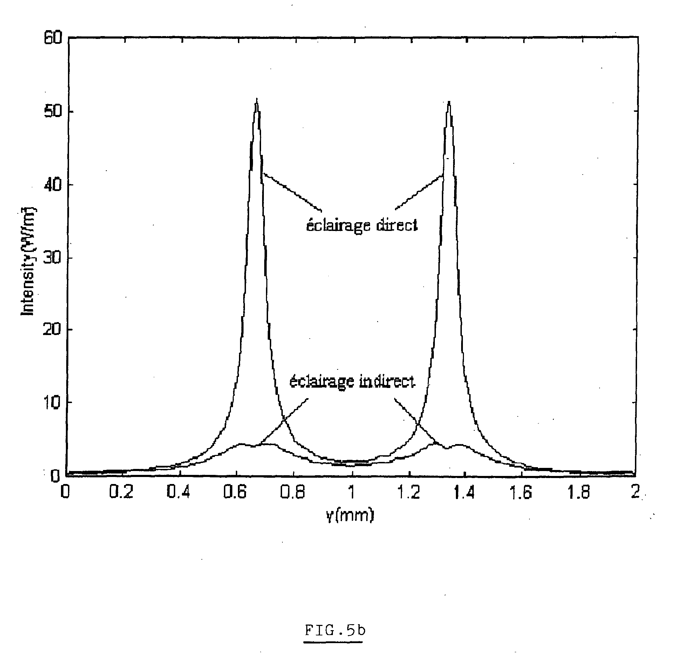

La figure 5.b montre des exemples de profils d'intensité lumineuse brute reçue par la grille de métal déployé en éclairage direct et indirect.

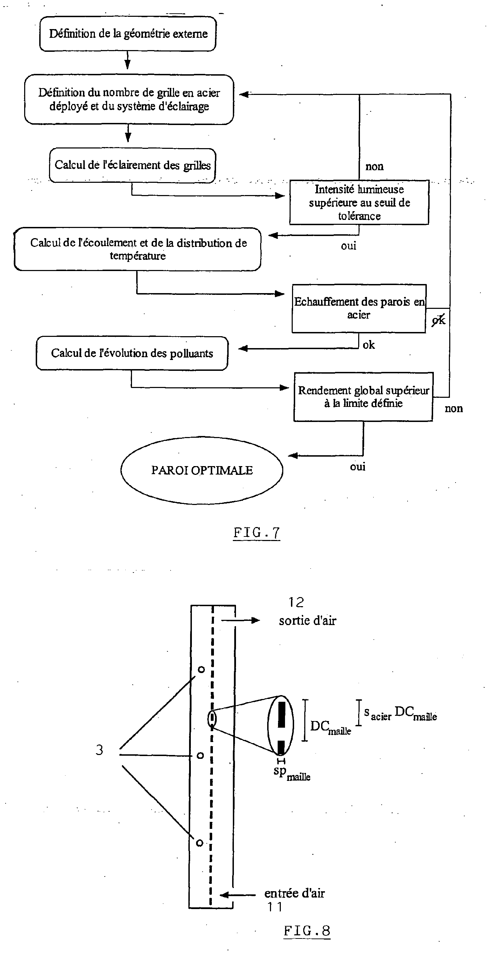

La figure 6 représente la carte d'intensité lumineuse utilisée dans le modèle de purification associé à la présente invention.

La figure 7 montre l'algorithme d'optimisation du dimensionnement de la paroi purificatrice selon la présente invention.

La figure 8 montre schématiquement un exemple de modèle bi-dimensionnel de paroi purificatrice composée d'une grille et de trois lampes.

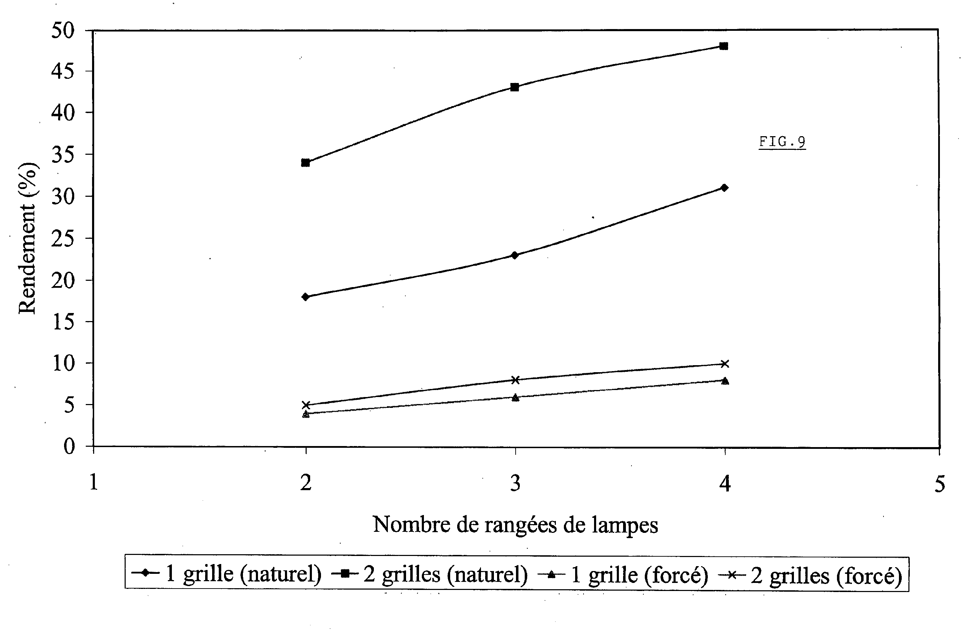

La figure 9 représente graphiquement le rendement de la paroi purificatrice selon la présente invention en fonction du nombre de grilles, du nombre de rangées de lampes et du type de circulation d'air (naturelle ou forcée).

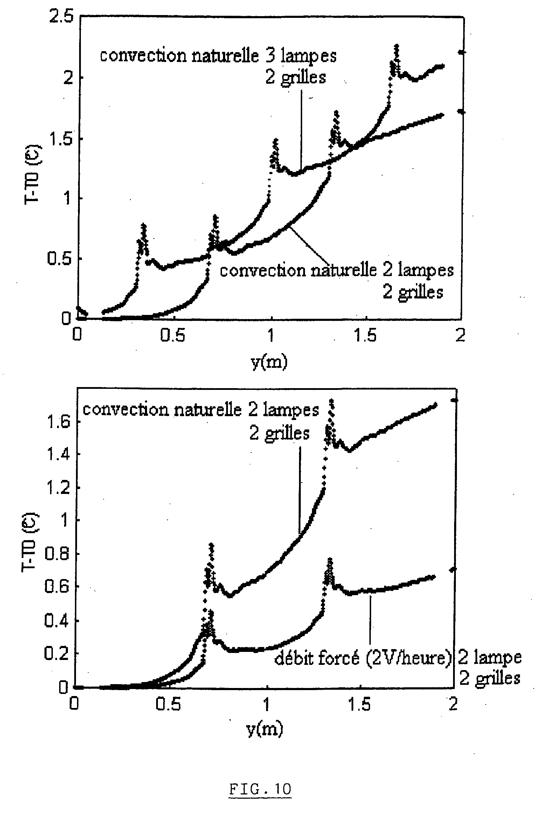

La figure 10 représente des profils de température sur les parements extérieurs de la paroi purificatrice en fonction du nombre de lampes UVA et du type d'écoulement considéré.

## Description d'une forme d'exécution préférée de l'invention

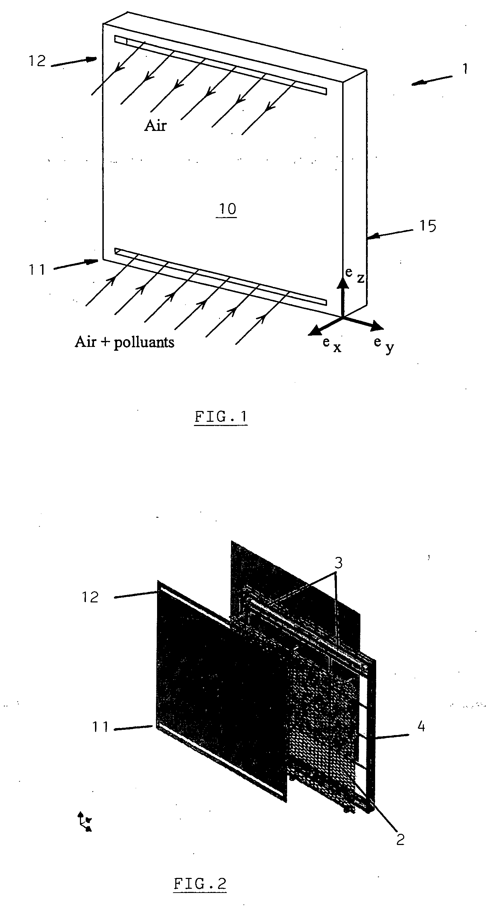

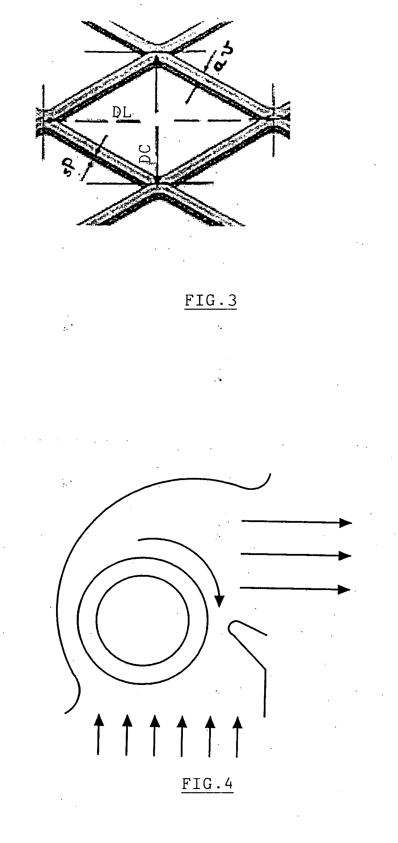

La paroi purificatrice d'air 1 représenté sur les figures 1 et 2 fonctionne sur le principe connu de la photocatalyse hétérogène au contact du dioxyde de titane. Cet appareil comprend une paroi active 1 travaillant en boucle ouverte qui comprend :

- une entrée d'air 11 située sur la partie inférieure de la face avant 10 de la paroi par laquelle le mélange d'air et de polluants éventuels pénètre à l'intérieur de l'appareil ;

- à l'intérieur de la paroi, une ou plusieurs grilles 2 en acier déployé, sur lesquelles est déposé le dioxyde de titane, éclairées par un dispositif d'éclairage composé de lampes UVA 3 pour l'activation du film de $TiO_2$ ;
- une ouverture 12 située sur la partie supérieure de la face avant 10 de la paroi par laquelle l'air, débarrassé de ses polluants, est éjecté. L'écoulement d'air est induit soit par des ventilateurs (convection ou circulation forcée), soit par l'augmentation de température due au système d'éclairage (circulation naturelle).

Pour dimensionner de manière optimale l'appareil de purification d'air selon l'invention, on a utilisé des modèles mathématiques et numériques, notamment pour décrire l'écoulement convectif turbulent et thermique induisant la circulation des polluants dans la paroi. Un modèle théorique de purification développé pour la circonstance et validé à l'aide de résultats expérimentaux a été introduit au niveau des conditions limites afin d'évaluer l'efficacité de l'appareil de purification du point de vue de la dépollution.

L'étude globale du design et du dimensionnement de la paroi doit ainsi prendre en compte les contraintes thermiques, acoustiques, structurelles et les phénomènes de purifications. L'étude menée se focalise principalement sur l'aspect thermique et sur la dépollution.

Dans le cadre du dimensionnement de la paroi, on doit introduire le calcul de l'écoulement, de la distribution de température et le processus de purification dans un algorithme global d'optimisation sans découplage. La raison de ce couplage apparaît clairement par la définition des critères de dimensionnement.

La paroi purificatrice 1 est placée dans un espace confiné, tel que des bureaux, salles de réunion, etc. La température ambiante dans cet espace est donc reliée à la température de la paroi en acier agissant comme condition limite. De plus, le contact direct potentiel entre les occupants de l'espace et la paroi nécessite le contrôle de la température superficielle de l'acier. Par conséquent, le premier critère d'optimisation est la minimisation de l'échauffement de la paroi.

Le second critère d'optimisation est le rendement du processus de purification. On doit maximiser le rendement global de la photocatalyse afin de diminuer le temps de purification de l'espace habité considéré.

Ces deux critères font apparaître une fonction d'optimisation qui possède un extremum. En effet, pour satisfaire au premier critère, il faut diminuer l'échauffement de la paroi par le système d'éclairage et à la limite, le faire tendre vers zéro. Pour le deuxième critère, il faut avoir un flux photonique suffisant pour que le rendement global du processus de purification soit maximal et par conséquent intensifier le système d'éclairage. L'optimum consiste à déterminer le système d'éclairage qui provoque un échauffement minimal en conservant un rendement de purification maximal.

Pour les calculs, les dimensions extérieures de la paroi 1 étant fixées à 2 m de hauteur ($H_{paroi}$), 2 m de largeur ($L_{paroi}$) et 10 cm d'épaisseur ($e_{paroi}$) , les trois paramètres sur lesquels on peut optimiser le dimensionnement sont : le nombre de grilles 2 de métal déployé, le nombre de lampes UVA 3 utilisées dans le système d'éclairage et l'utilisation de ventilateur(s) 5 pour forcer l'écoulement (voir par exemple Fig. 5).

Comme représenté à la figure 2, la paroi activé en acier utilisée comprend un ensemble d'éléments possédant chacun une fonction particulière qui permet d'obtenir un système de purification d'air par photocatalyse optimal : la structure externe en acier, essentiellement les faces avant 10 et arrière 15, les grilles 2 de métal déployé sur lesquelles un film de $TiO_2$ est déposé, le système d'éclairage comprenant des lampes UVA 3 fixées à une ossature en métal 4. L'ensemble est complété par des ventilateurs 5 et une boîte électrique alimentant en énergie toute la paroi (non représentés).

## 1. *La structure externe*

La structure externe de la paroi active 1 est constituée de plaques en acier. Les propriétés de surface de ces plaques jouent un rôle capital au niveau de l'éclairement du dioxyde de titane.

Afin d'obtenir un rendement optimal de la photocatalyse au contact du dioxyde de titane, il faut que toutes les grilles en métal déployé soient illuminées avec une lumière ultraviolette provenant des différentes lampes constituant le système d'éclairage. Pour ce faire, les surfaces internes des parements en acier de la structure externe doivent avoir un facteur de réflexion maximal pour les longueurs d'onde inférieures à la longueur d'onde de référence du dioxyde de titane anatase, à savoir 387 nm. On doit donc utiliser un acier dont le facteur de réflexion dans l'ultra-violet est élevé (supérieur à 90%), par exemple de l'acier inoxydable recuit brillant.

Selon la forme d'exécution préférée, mise en oeuvre, la face avant 10 de la structure externe possède deux ouvertures de 1,9 m de long sur 5 cm de haut. L'ouverture 11 située dans le bas de la face avant 10 permet à l'air de pénétrer dans la paroi afin de subir la photocatalyse hétérogène au contact du dioxyde de titane et donc de se purifier.

L'ouverture 12 située sur la partie haute de la face avant 10 permet de réinjecter l'air purifié dans l'espace que l'on désire traiter.

Les dimensions des ouvertures sont déterminées en fonction de plusieurs paramètres : la quantité d'air traitée, le temps de résidence de l'air dans la paroi et les vitesses de l'air aux entrée et sortie de la paroi.

Pour maximiser la quantité d'air traitée par la paroi active, il faut que la surface de l'ouverture d'entrée 11, située dans le bas de la face avant, soit la plus grande possible. On a donc opté pour une longueur de 1,9 m, ce qui satisfait les contraintes mécaniques au niveau de la résistance de la face avant et les contraintes sur la quantité d'air à traiter. La longueur de l'ouverture de sortie 12 est égale à la longueur de l'ouverture d'entrée 11 car pour obtenir un rendement maximum de purification, il faut que l'écoulement d'air dans la paroi soit uniforme horizontalement, ce qui est précisément obtenu avec des longueurs d'entrée et sortie égales.

Afin de maximiser le temps de résidence de l'air dans la paroi active, il est essentiel de placer les entrée et sortie à une distance maximale. On a donc placé l'ouverture d'entrée à 5 cm du bas de la paroi active et l'ouverture de sortie à 5 cm du haut de celle-ci.

Selon les normes IAQ, les vitesses d'air moyennes acceptables dans les bâtiments sont de l'ordre de 20 à 30 cm/s. De plus, le taux de renouvellement de l'air pour une personne est de l'ordre de 30 $m^3$/h. Pour satisfaire ces deux conditions, la hauteur des ouvertures 11,12 est définie à 5 cm.

*2. Les grilles en métal déployé*

Les grilles en métal déployé 2 constituent le support du semi-conducteur utilisé pour le processus de photocatalyse hétérogène. Dans l'étude menée, on a considéré le dioxyde de titane majoritairement en phase anatase.

Le métal déployé est défini comme une matrice dont l'élément fondamental est la maille 6 (voir figure 3).

Cette maille 6 est définie par quatre paramètres : la diagonale longue ($DL_{maille}$), la diagonale courte ($DC_{maille}$) , la largeur de la lanière ($av_{maille}$) et l'épaisseur ($sp_{maille}$). A partir des trois premiers paramètres, on peut définir la surface de métal contenue dans une maille ($S_{acier}$) :

$$S_{acier} = [av_{maille} \sqrt{(\frac{DL_{maille}}{2})^2 + (\frac{DC_{maille}}{2})^2} - \frac{- av_{maille}^2}{2 \sin(2 arctg(\frac{DC_{maille}}{DL_{maille}}))}] \tag{1.1}$$

Pour définir les quatre paramètres du métal déployé, nous devons tenir compte de plusieurs paramètres : la surface totale du film de dioxyde de titane et l'éclairement de cette surface grâce au système d'éclairage.

Pour obtenir un rendement de purification optimal, il faut que la surface totale du film $TiO_2$ soit maximale. Lorsque le $TiO_2$ est déposé sur le métal déployé, la surface recouverte de $TiO_2$ est égale à la surface de métal ($S_{acier}$). Cette égalité entre les deux surfaces est exacte s'il n'y a pas de dioxyde de titane déposé sur l'épaisseur de la maille 6. En nous basant sur la méthode de dépôt industrielle qui devra être envisagée, nous considérons que cette hypothèse est valable. En effet, pour une application industrielle, le dépôt de dioxyde de titane se fera sur une tôle en acier continue avant qu'elle ne soit déployée, ce qui implique donc l'absence de dioxyde de titane sur l'épaisseur de la maille.

De plus, pour que le processus de photocatalyse hétérogène au contact du dioxyde de titane soit maximal, il faut que toute la surface du semi-conducteur soit éclairée avec la lumière UVA provenant du système d'éclairage. La paroi active pouvant comporter plusieurs grilles de métal déployé, il faut dès lors que la lumière puisse atteindre les deux faces de chaque grille. Pour ce faire, le rapport entre la surface physique de la maille ($S_{acier}$) et la surface de la maille ($S_{maille}$) doit être minimal :

$$S_{acier} = \frac{S_{acier}}{S_{maille}} = \frac{4}{DL_{maille}DC_{maille}} S_{acier} \tag{1.2}$$

Afin de rencontrer ces deux impératifs, nous avons opté pour un rapport $s_{acier}$ de l'ordre de 1/3.

A partir du catalogue de métal déployé de la société MDB (Métal Déployé Belge S.A., Groupe Arcelor), on a sélectionné trois métaux acceptables dont les références sont 62.25.43.30, A28.15.21.10, A28.15.25.15, ce qui correspond respectivement aux dimensions suivantes ($DL_{maille}$ = 62 mm, $DC_{maille}$ = 25 mm, $av_{maille}$ = 4,3 mm, $sp_{maille}$ = 3 mm), ($DL_{maille}$ =28 mm, $DC_{maille}$ = 15 mm, $av_{maille}$ = 2,5 mm, $sp_{maille}$ = 1mm), ( $DL_{maille}$ =28 mm, $DC_{maille}$ =15 mm, $av_{maille}$ =2,5 mm, $sp_{maille}$ =1,5 mm).

Le dernier critère de sélection du type de métal déployé est fonction de sa rigidité structurelle. Les grilles de métal déployé sont placées verticalement dans la paroi active. Afin d'éviter d'encombrer la paroi avec des renforts internes, le métal déployé utilisé doit pouvoir être maintenu vertical avec uniquement des fixations situées sur le pourtour de la grille.

Ce critère nous permet de finaliser notre choix sur le métal déployé 62.25.43.30 car il possède une épaisseur suffisante pour vérifier le critère de rigidité.

*3. Le système d'éclairage*

Le système d'éclairage est constitué d'un ensemble de lampes UVA de 25 W type CLEO 2 (Philips). Ce type de lampe fournit une puissance de 4,3 W en UVA, c'est-à-dire pour des longueurs d'onde comprises entre 320 et 400 nm. Elles fournissent également un rayonnement UVB dont le rapport avec les UVA est de 1,2%. Leur dimensions sont : une longueur de 516,9 mm et un diamètre de 16 mm.

Les lampes sont disposées par série de trois placées sur la largeur de la paroi ($L_{paroi}$). Le nombre de séries est déterminé lors du calcul du dimensionnement de la paroi. Les critères permettant de déterminer le nombre de séries sont : obtenir un échauffement minimal de l'air et de la structure externe de la paroi et obtenir un seuil minimal d'éclairement de toutes les surfaces des grilles de métal déployé sur lesquelles le dioxyde de titane est déposé.

*4. Les ventilateurs*

Les ventilateurs 5 assurent la circulation de l'air à travers la paroi active. Le débit d'air induit par les ventilateurs doit répondre aux normes de renouvellement d'air. Les normes recommandent un renouvellement d'air de l'ordre de 30 m$^3$/heure/personne pour les espaces habités.

L'adaptation du débit d'air peut être envisagé en fonction du type d'espace à purifier.

Nous utilisons des ventilateurs tangentiels à 90°, c'est-à-dire des ventilateurs permettant de modifier la direction de l'écoulement de 90°, de la marque Ziehl-Abegg et de type QR 06-GKM70BP. Ce type de ventilateur permet d'atteindre des débits importants de l'ordre de 550 m$^3$/h.

Le débit est adapté à l'aide d'un potentiomètre régulant la puissance électrique fournie au moteur.

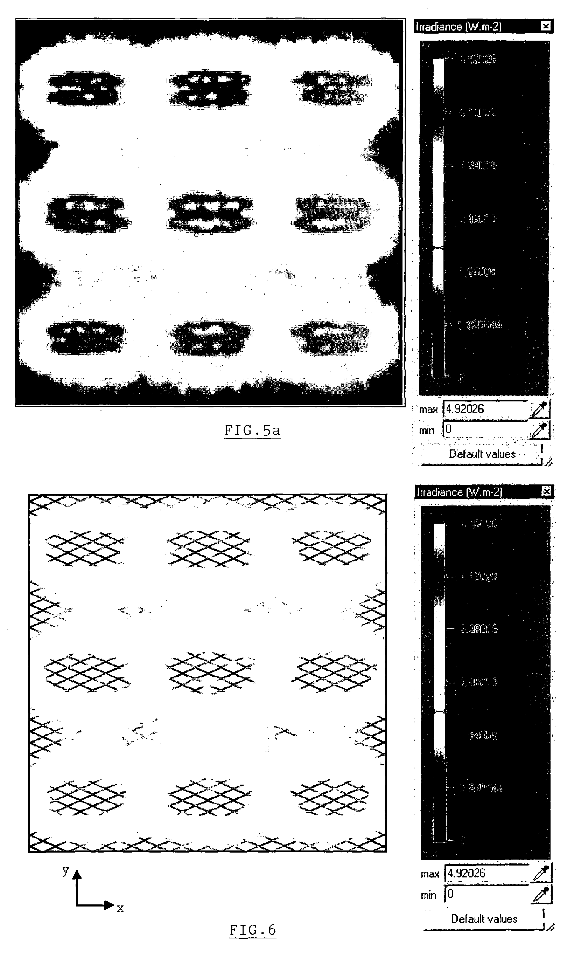

La figure 4 montre la modification de la direction d'écoulement dans un ventilateur tangentiel à 90°.

Les ventilateurs sont placés en haut de la paroi active afin d'obtenir un écoulement uniforme dans toute la paroi.

*5. Le boîtier électrique*

Le fonctionnement de la paroi active nécessite une source d'électricité afin d'alimenter le système d'éclairage et les ventilateurs. Afin d'éviter la présence d'une fiche électrique pour chaque lampe et pour chaque ventilateur, on installe un boîtier électrique dans un coin inférieur. Ce boîtier électrique, alimenté par une seule fiche électrique provenant de l'extérieur, fournit l'énergie nécessaire à tous les composants électriques de la paroi.

*6. Ecoulement et conditions limites*

Pour la modélisation des écoulements, on utilise les équations de Navier-Stokes incompressibles et stationnaires. Les effets thermiques sur l'écoulement sont également introduits dans le modèle par l'approximation de Boussinesq. Le nombre de Reynolds, défini par le débit d'air dans la paroi et la surface d'entrée, est suffisamment grand pour justifier l'utilisation d'un modèle de turbulence.

Les faibles vitesses d'écoulement, de l'ordre de 1 m/s, ainsi que le faible échauffement de la paroi vérifient les hypothèses nécessaires à l'utilisation de l'approche incompressible et l'approximation de Boussinesq.

6.1 Conditions limites dynamiques

Pour l'appareil de purification, nous devons imposer un débit d'air à l'entrée.

A une entrée d'air, nous devons imposer deux conditions limites dynamiques. Nous introduisons le débit d'air $Q_{air}$, que nous ferons varier lors de l'étude numérique, et la direction de l'écoulement, que nous considérons perpendiculaire à la surface d'entrée.

A la sortie, une seule condition limite est requise. Nous imposons la valeur de la pression de référence.

6.2 Conditions limites turbulentes

A l'entrée de l'appareil de purification, nous devons introduire une condition pour l'énergie cinétique turbulente (k) et la dissipation de l'énergie cinétique turbulente ($\varepsilon$). Ces conditions sont très difficiles à évaluer car elles dépendent de l'écoulement en amont de l'entrée de l'appareil, écoulement qui n'est pas modélisé dans les simulations numériques.

Pour définir les conditions limites, nous raisonnons en fonction de l'objectif de l'étude. Dans cette étude concernant le dimensionnement de la paroi purificatrice, nous devons évaluer le rendement de l'appareil au niveau de la purification de l'air. On peut montrer que le brassage de l'air favorise le processus de purification car il assure un apport optimal du polluant vers la surface du semiconducteur. Ce brassage d'air est principalement caractérisé par le niveau de turbulence : plus la turbulence est importante, meilleure est l'homogénéisation du polluant.

Afin d'étudier le cas le plus critique, c'est-à-dire le cas où le brassage est minimal, nous considérons qu'à l'entrée de l'appareil de purification l'écoulement est laminaire. La turbulence est uniquement générée à l'intérieur de la paroi. Les conditions limites à imposer à l'entrée pour l'énergie cinétique turbulente et sa dissipation deviennent donc très simples. Nous annulons l'énergie cinétique turbulente ainsi que sa dissipation.

6.3 Conditions limites thermiques

Pour les conditions limites thermiques, nous définissons quatre conditions différentes : la condition limite à l'entrée de l'appareil de purification, la condition limite sur les lampes, la condition limite sur les faces internes de la paroi et la condition limite sur les différentes faces des grilles de métal déployé contenant le dioxyde de titane.

L'expression générale d'une condition limite thermique fait intervenir le flux convectif, le flux radiatif, le flux conductif et le flux externe. Cette expression générale se simplifie en fonction de la surface considérée.

A) L'entrée de l'appareil de purification.

Cette condition limite est la plus simple. Elle va permettre d'établir le niveau de température moyen dans la paroi purificatrice. Pour ce faire, nous imposons une température de 20 °C.

$$T_{entrée} = 20°\,C \tag{1.3}$$

B) Les lampes.

L'apport énergétique est assuré par un système de lampes de type CLEO de 25 W assurant une puissance lumineuse de 4,3 W. La condition limite sur chaque lampe s'écrit :

$$k_0 \frac{d}{d\vec{n}} \left. T \right|_{plaque} + q''_{radiatif} + q''_{lampes} = 0 \tag{1.4}$$

où le flux thermique des lampes est donné par l'expression :

$$q''_{lampes} = \frac{P}{S_{lampes}} = \frac{4,3}{2\pi R_{lampe} L_{lampe}} = 147 \mathrm{W}/m^2 \tag{1.5}$$

où $R_{lampe}$ est le rayon de la lampe (8 mm) et $L_{lampe}$ est la longueur de la lampe (517 mm).

C) Les faces internes de la paroi.

La condition limite thermique sur la paroi est très compliquée car nous devons tenir compte des échanges conductifs à l'intérieur de la plaque en acier inoxydable recuit brillant, des échanges convectifs et radiatifs à l'extérieur de la paroi. Ce type de données ne peut être précisément défini.

Deux solutions peuvent être envisagée : utiliser des coefficients d'échange convectif et radiatif externe moyen ou considérer le cas le plus défavorable pour le dimensionnement de l'appareil de purification. Nous optons pour la deuxième solution. En effet, si nous pouvons obtenir une paroi purificatrice avec un échauffement minimal dans les conditions les plus défavorables, nous sommes certains d'englober tous les cas.

Analysons le cas le plus défavorable. Lorsque le système d'éclairage, composé des lampes UV de type CLEO, fonctionne dans la paroi, les grilles de métal déployé ainsi que les plaques en acier inoxydable recuit brillant s'échauffent. La face avant de la paroi est en contact direct avec la température de l'air ambiant dans l'espace voisin. Il existe donc un transfert thermique de la face avant vers l'espace voisin. L'espace voisin tend donc à faire diminuer la température de la face avant. Le cas le plus défavorable serait de considérer que l'air de l'espace voisin atteint directement la valeur de la température de la face avant : cette condition est une condition d'adiabaticité. Nous ne prenons pas en compte la possibilité d'un éclairement externe de la face avant qui logiquement ferait augmenter la température.

Nous utilisons donc la condition d'adiabaticité, c'est-à-dire de considérer la somme du flux conductif dans la face avant et des flux convectif et radiatif externes comme nulle.

La condition limite s'écrit donc :

$$k_0 \, \frac{d}{d\vec{n}} \, T|_{plaque} + q''_{radiatif} + \alpha_{inox} q''_{lumineux} = 0 \qquad (1.6)$$

où $q''_{lumineux}$ est le flux lumineux reçu par la surface en provenance des lampes et $\alpha_{inox}$ est le coefficient d'absorptivité de l'acier inoxydable recuit brillant.

Le coefficient d'absorptivité de l'acier inoxydable recuit brillant est de l'ordre de 0,15 dans l'infrarouge et monte à une valeur de l'ordre de 0,3 dans l'ultraviolet.

D) Les grilles de métal déployé.

Les grilles en métal déployé sont complètement comprises dans le domaine de calcul. Nous utilisons donc un type de condition limite couplée, avec un apport supplémentaire dû à l'apport énergétique par le système d'éclairage.

*7. Polluants et modèle théorique de purification*

L'appareil de purification, la paroi purificatrice, doit fonctionner pour des mélanges complexes constitués d'une gamme étendue de polluants. Les polluants sont regroupés sous le nom générique de composés volatiles organiques (VOC). Des normes régissant les taux de pollution dans les bâtiments imposent des limites en fonction de chaque polluant mais également sur la quantité totale de composés volatiles organiques (TVOC). Les concentrations moyennes admises sont de l'ordre de 200 - 500 $\mu g/m^3$. Ce niveau de concentration correspond à des valeurs inférieures au $ppm_{volumique}$ pour chaque polluant.

Nous répertorions les principaux polluants en 6 catégories : les alcanes, les alcools, les aldéhydes, les cétones, les aromatiques et les terpènes.

La première catégorie de polluants est celle des alcanes. Les alcanes sont des molécules uniquement constituées d'un chaîne carbonée sur laquelle des atomes d'hydrogène sont greffés (Tableau 1).

Tableau 1

| *Alcane* | *Formule* | $C_A$ ($\mu g/m^3$) | $MW_A$($g/mol$) | $C_A$ ($ppb_{volumique}$) |
|---|---|---|---|---|
| Hexane | $C_6H_{14}$ | 9.85 | 86.2 | 2.56 |
| Heptane | $C_7H_{16}$ | 12 | 100.23 | 2.68 |
| Octane | $C_8H_{18}$ | 10.1 | 114.26 | 1.98 |
| Nonane | $C_9H_{20}$ | 8.5 | 128.29 | 1.48 |
| Decane | $C_{10}H_{22}$ | 15.7 | 142.32 | 2.47 |
| Undecane | $C_{11}H_{24}$ | 20.85 | 156.35 | 2.98 |
| Dodecane | $C_{12}H_{26}$ | 12.05 | 170.38 | 1.58 |
| Tridecane | $C_{13}H_{28}$ | 3.8 | 184.41 | 0.46 |
| Tetradecane | $C_{14}H_{30}$ | 10.3 | 198.44 | 1.16 |

La concentration totale en alcane, obtenue par sommation de la concentration moyenne de chaque composé, est de 103 $\mu g/m^3$.

Dans la catégorie générale des alcanes, il existe des composés dont la chaîne carbonée est cyclique. Ce sont les cycloalcanes (Tableau 2).

Tableau 2

| *Cycloalcane* | *Formule* | $C_A$ ($\mu g/m^3$) | $MW_A$ ($g/mol$) | $C_A$ ($ppb_{volumique}$) |
|---|---|---|---|---|
| Cyclohexane | $C_6H_{12}$ | 5.6 | 84.16 | 1.49 |
| Methylcyclohexane | $C_7H_{14}$ | 6.6 | 98.19 | 1.50 |

Tableau 2   (suite)

| Cycloalcane | Formule | $C_A$ ($\mu g/m^3$) | $MW_A$ (g/mol) | $C_A$ (ppb$_{volumique}$) |
|---|---|---|---|---|
| Methylcyclopentane | $C_6H_{12}$ | 1.6 | 84.16 | 0.42 |

Les cycloalcanes représentent une faible partie des composés appartenant à cette catégorie des alcanes. Leur concentration totale est de 14 $\mu g/m^3$.

La seconde catégorie des composés volatiles organiques que nous avons répertoriés est celle les alcools (Tableau 3).

Tableau 3

| Alcool | Formule | $C_A$($\mu g/m^3$) | $MW_A$ (g/mol) | $C_A$ (ppb$_{volumique}$) |
|---|---|---|---|---|
| 1-Butanol | $C_4H_{10}O$ | 5.5 | 74.12 | 1.66 |
| 2-Butoxyethanol | $C_6H_{14}O_2$ | 56 | 118.17 | 10.62 |
| 1-Hexanol-2-Ethyl | $C_8H_{18}O$ | 4.1 | 130.23 | 0.7 |
| Phénol | $C_6H_6O$ | 3.4 | 94.11 | 0.8 |

Les quatre principaux polluants appartenant à cette catégorie ont des concentrations individuelles telles que leur totalité représente 69 $\mu g/m^3$.

La troisième catégorie est caractérisée par les composés aldéhydes. Ce sont ces composés dont les concentrations individuelles sont les plus élevées, en particulier le formaldéhyde et l'acétaldéhyde (Tableau 4).

Tableau 4

| Aldéhyde | Formule | $CA$($\mu g/m^3$) | $MWA$(g/mol) | $CA$ (ppb$_{volumique}$) |
|---|---|---|---|---|
| Formaldéhyde | $CH_2O$ | 77.22 | 30.03 | 57.63 |
| Acétaldéhyde | $C_2H_4O$ | 28.28 | 44.05 | 14.38 |
| Propionaldéhyde | $C_3H_6O$ | 3.86 | 58.08 | 1.48 |
| Crotonaldéhyde | $C_4H_6O$ | 2.52 | 70.09 | 0.8 |
| Butyraldéhyde | $C_4H_8O$ | 2.84 | 72.11 | 0.88 |
| Benzaldéhyde | $C_6H_7O$ | 2.1 | 106.12 | 0.44 |

Les concentrations de chaque composé de cette catégorie sont telles que leur concentration totale est de 116 $\mu g/m^3$.

La quatrième catégorie est celle des cétones. Les trois composés répertoriés dans le Tableau 5 représentent une concentration totale de 30,8 $\mu g/m^3$.

Tableau 5

| Cétone | Formule | $C_A$($\mu g/m^3$) | $MW_A$(g/mol) | $C_A$ (ppb$_{volumique}$) |
|---|---|---|---|---|
| Acétone | $C_3H_6O$ | 26 | 58.08 | 10.03 |
| 2-Butanone | $C_4H_8O$ | 3.4 | 72.11 | 1.05 |
| 4-Methyl-2-pentanone | $C_6H_{12}O$ | 1.4 | 100.16 | 0.3 |

Les aromatiques sont les composés appartenant à la cinquième catégorie. Cette catégorie possède également un composé dont la concentration est élevée, le toluène (Tableau 6).

Tableau 6

| Aromatique | Formule | $C_A$($\mu g/m^3$) | $MW_A$(g/mol) | $C_A$ (ppb$_{volumique}$) |
|---|---|---|---|---|
| Benzène | $C_6H_6$ | 4.43 | 78.12 | 1.27 |
| Toluène | $C_7H_8$ | 62.32 | 92.15 | 15.15 |

Tableau 6   (suite)

| Aromatique | Formule | $C_A(\mu g/m^3)$ | $MW_A(g/mol)$ | $C_A$ (ppb$_{volumique}$) |
|---|---|---|---|---|
| o-Xylène | $C_8H_{10}$ | 3.93 | 106.17 | 0.82 |
| Styrène | $C_8H_8$ | 0.92 | 104.15 | 0.19 |
| Ethylbenzène | $C_8H_{10}$ | 5.99 | 106.17 | 1.26 |
| 2-Ethyltoluène | $C_9H_{12}$ | 2.3 | 120.19 | 0.42 |
| 3-Ethyltoluène | $C_9H_{12}$ | 3.7 | 120.19 | 0.69 |
| 4-Ethyltoluène | $C_9H_{12}$ | 1.9 | 120.19 | 0.35 |
| 1,2,4-Trimethylbenzène | $C_9H_{12}$ | 0.7 | 120.19 | 0.13 |

La concentration totale des composés aromatiques est de 86 $\mu g/m^3$.

La dernière catégorie englobe les composés terpènes (Tableau 7). La concentration totale est de 76.8$\mu g/m^3$.

Tableau 7

| Terpène | Formule | $C_A(\mu g/m^3)$ | $MW_A(g/mol)$ | $C_A$ (ppb$_{volumique}$) |
|---|---|---|---|---|
| Pinène | $C_{10}H_{16}$ | 18.3 | 136.23 | 3.01 |
| Limonène | $C_{10}H_{16}$ | 58.5 | 136.23 | 9.62 |

La concentration totale en composés volatiles organiques est de 420 $\mu g/m^3$.

Cette liste non exhaustive des polluants montrent que leur concentration moyenne est inférieure au ppm$_{volumique}$. Lors de l'analyse des résultats expérimentaux sur les mélanges complexes à basses concentrations, nous avons observé que chaque polluant subit le processus de purification par photocatalyse au contact du dioxyde de titane comme s'il se trouvait seul dans la boîte d'essai. Nous utilisons cette propriété pour introduire tous les composés précédemment cités dans un algorithme de dimensionnement de l'appareil de purification. En utilisant cette propriété, nous définissons un polluant test A dont la concentration initiale est la concentration totale de VOC, c'est-à-dire 420 $\mu g/m^3$.

Nous avons pu définir précisément les paramètres dans le modèle précité de purification dans le cas de la purification de l'acétaldéhyde. Ces paramètres, le rendement quantique et la concentration de référence, dépendent du flux photonique reçu par le dioxyde de titane. A partir des trois valeurs des paramètres, déterminées respectivement pour un flux photonique de $1,66.10^{-4}$ ,de $7,26.10^{-5}$ et de $2,56.10^{-5}$ einstein/m$^2$/s, nous définissons une fonction d'interpolation pour le rendement quantique et la concentration de référence. Ces fonctions d'interpolation permettent d'obtenir la valeur des paramètres de purification en fonction du flux photonique. La précision des valeurs est assurée tant que le flux photonique incident est compris entre $1.66\ 10^{-4}$ et $2.56\ 10^{-5}$ einstein/m$^2$/s.

La connaissance précise des paramètres de purification pour l'acétaldéhyde fait de ce composé le polluant test permettant d'introduire tous les composés VOC cités précédemment dans les listes.

Nous dimensionnons donc l'appareil de purification selon l'invention en considérant une atmosphère polluée uniquement avec de l'acétaldéhyde dont la concentration est de 420 $\mu g/m^3$, ce qui correspond à 213 ppb$_{volumique}$. Afin d'assurer une marge de sécurité au dimensionnement, nous considérerons que la concentration d'acétaldéhyde est de 1 ppm$_{volumique}$.

Lors de l'analyse des résultats expérimentaux, nous n'avons jamais constaté l'apparition de produits intermédiaires dans le processus de décomposition de l'acétaldéhyde au contact du film de dioxyde de titane déposé par spray. La seule réaction de décomposition que nous considérons est donc la réaction de décomposition de l'acétaldéhyde par les radicaux d'hydroxyles.

L'utilisation de notre modèle théorique de purification comme condition limite dans le calcul global de dispersion de l'acétaldéhyde dans la paroi purificatrice nécessite la détermination des paramètres de purification, que nous obtenons par les fonctions d'interpolation sur des valeurs déterminées par ailleurs (non publié), la détermination des coefficients stoechiométriques, que nous obtenons grâce à l'équation de décomposition de l'acétaldéhyde et la détermination du flux photonique reçu par la surface de dioxyde de titane.

7.1 Flux photonique

L'évaluation du flux photonique est une étape importante dans l'algorithme d'optimisation. Pour ce calcul, nous utilisons un logiciel de calcul optique, par exemple le logiciel SPEOS (Optis, France). Ce logiciel détermine des cartes d'éclairement, des cartes de flux lumineux, en calculant la propagation des rayons lumineux suivant les lois de la physique. Ces cartes permettent de définir la fonction spatiale de distribution de l'intensité lumineuse sur une surface donnée ($F_{SPEOS}$) :

$$F_{SPEOS}(x,y) = \int\limits_{spectre\_total} I(\lambda, x, y)d\lambda \quad (1.7)$$

où :

- x, y sont les coordonnées spatiales attachées à la surface ;
- *spectre_total* est le spectre d'émission total des lampes utilisées ;
- I est l'intensité lumineuse ($W/m^2/nm$) ;
- $\lambda$ est la longueur d'onde du rayonnement (nm).

Pour notre modèle théorique de purification, nous devons déterminer le flux photonique c'est-à-dire le nombre de photons qui atteint la surface éclairée en un point de coordonnée (x,y) par unité de temps.

Pour relier les données obtenues par les cartes d'éclairement et le nombre de photons ($N_{h\nu}$), nous faisons l'hypothèse suivante : l'intensité lumineuse I ($\lambda$,x,y) peut être séparée en trois contributions, la première est la norme de l'intensité lumineuse $|I|$ , la seconde dépend uniquement de la position spatiale du point de la surface considérée $\bar{I}_s$ (x,y) et la troisième représente la dépendance spectrale $\bar{I}_\lambda(\lambda)$. Ces deux dernières grandeurs sont normalisées. On a :

$$I(\lambda,x,y)= |I|\bar{I}_s(x,y)\bar{I}_\lambda(\lambda) \; ( \tag{1.8}$$

En introduisant cette définition de l'intensité lumineuse (1.8), nous réécrivons l'équation du flux photonique reçu au point x,y de la surface éclairée :

$$\frac{dN_{h\nu}(x,y)}{dt} = \frac{|I|\bar{I}_s(x,y)}{N_{Avogadro}}\left[\int\limits_0^{\lambda_{gap}} \frac{\bar{I}_\lambda(\lambda)}{E_{h\nu}(\lambda)}d\lambda\right] \quad (1.9),$$

où E(hν) = hν = h.c/$\lambda$.

L'intégrale sur les longueurs d'onde absorbables par le semiconducteur peut être évaluée lorsque l'on connaît le spectre d'émission de la source (généralement fourni par la société produisant la lampe). Pour les lampes de type CLEO de la firme Philips, nous constatons qu'il y a une diminution de l'intensité lumineuse en fonction de la distance entre le système d'éclairage et la surface éclairée. Lorsque les spectres sont normalisés, nous obtenons le spectre d'émission de la lampe fourni par le fabricant.

Nous définissons donc un flux photonique normalisé ($F_{h\nu,0}$), que l'on peut calculer uniquement à partir des données du fabricant et qui est constant sur toute la surface éclairée :

$$\overline{F}_{h\nu,0} = \frac{1}{N_{Avogadro}}\left[\int\limits_0^{\lambda_{gap}} \frac{\bar{I}_\lambda(\lambda)}{E_{h\nu}(\lambda)}d\lambda\right] = 9{,}695x10^{-14} \quad (1.10)$$

Introduisons également la relation (1.8) dans l'expression (1.7), nous obtenons l'expression de la fonction $F_{SPEOS}$ suivante :

$$F_{SPEOS}(x,y) = |I| \bar{I}_s(x,y) \int_{spectre\_total} \bar{I}_\lambda(\lambda)d\lambda \quad (1.11)$$

Dans cette expression (1.11), nous obtenons de nouveau un terme qui peut être directement calculé à partir des données du fabricant de la source lumineuse :

$$\bar{I}_{source,0} = \int_{spectre\_total} \bar{I}_\lambda(\lambda)d\lambda = 3,909x10^{-8} \quad (1.12)$$

En introduisant les expressions (1.10, 1.11, 1.12) dans le calcul du nombre de photons reçus par la surface éclairée par unité de temps (1.9), nous obtenons la formule :

$$\frac{DN_{hv}(x,y)}{dt} = F_{SPEOS}(x,y)\frac{\bar{F}_{hv,0}}{\bar{I}_{source,0}} \tag{1.13}$$

(où $\bar{F}_{hv,0} / \bar{I}_{source,0}$ = 2,48.10$^{-6}$).

En utilisant cette formule (1.13), les cartes d'éclairement fournies par le logiciel SPEOS ($F_{SPEOS}(x,y)$) ainsi que les données spectrales de la source lumineuse, nous pouvons calculer le flux photonique reçu par chaque point de la surface du catalyseur.

### 7.2 Cartes d'éclairement

Dans le paragraphe précédent, nous avons montré qu'à partir d'une carte d'éclairement obtenue avec le logiciel SPEOS et des caractéristiques spectrales des sources lumineuses, nous avons toutes les données utiles pour notre modèle de purification.

Pour obtenir les cartes d'éclairement, nous introduisons dans le logiciel SPEOS un modèle tridimensionnel de la paroi purificatrice. Comme pour les modèles géométriques utilisés lors des simulations d'écoulement, nous déterminons les éléments essentiels, les éléments importants et les éléments négligeables. Néanmoins, les éléments définis dans chacune des trois catégories sont différents de ceux définis dans le cadre du calcul CFD *(Computational Fluid Dynamic)*. Les objectifs des simulations CFD et avec le logiciel SPEOS étant différents, il est normal de répertorier les éléments des trois catégories différemment.

Dans les éléments essentiels, c'est-à-dire les éléments dont les caractéristiques géométriques ainsi que leurs positions spatiales ne peuvent différer de la réalité, nous considérons les lampes UVA de type CLEO 25 W et les faces avant et arrière de la paroi purificatrice.

Dans les éléments importants, c'est-à-dire les éléments devant être pris en compte dans le modèle géométrique mais pouvant être décrit avec une géométrie et une position spatiale différentes de la réalité, nous considérons les grilles de purification. L'épaisseur de la grille, de 3 mm, ne joue pas un rôle primordial dans le calcul de l'éclairement. Les ombres que l'épaisseur de la grille peut provoquer peuvent être négligées. Nous considérons donc que les grilles sont des surfaces bi-dimensionnelles planes.

Dans les éléments négligeables, c'est-à-dire les éléments dont l'influence sur l'objectif de la simulation est négligeable, nous considérons les ventilateurs, les faces supérieures et inférieures ainsi que les cotés verticaux de la paroi, la boîte électrique contenant l'alimentation des différents systèmes électriques,... Nous négligeons les ventilateurs, la boîte électrique,... car les matériaux qui les composent ont un coefficient de réflexion dans la gamme des UVA très petit, ce qui signifie que la quantité d'énergie redirigée vers les surfaces des grilles recouvertes de dioxyde de titane est négligeable. Pour les faces supérieure, inférieure et latérales de la paroi, nous considérons que leur influence sur l'éclairement des grilles, qui leur sont perpendiculaires, est également négligeable.

Après avoir définis les éléments de notre modèle géométrique, nous introduisons les paramètres nécessaires à la simulation. Toutes les surfaces sont caractérisées par des propriétés physiques relatives au domaine de la lumière : le type de réflexion lumineuse à considérer, lambertienne, gaussienne ou autres,..., fonction de l'état de surface du matériau ; la dépendance spectrale de la réflectivité ; etc. Pour les sources lumineuses, nous introduisons leurs spectres d'émission ainsi que leurs puissances.

Les simulations à l'aide du logiciel SPEOS doivent nous fournir la quantité d'énergie reçue par chaque point de chaque grille de métal déployé. Pour obtenir ces informations, nous devons effectuer un post-traitement sur les cartes d'éclairement. En effet, les cartes d'éclairement sont de simples surfaces bi-dimensionnelles planes qui "comptent" le nombre de rayons lumineux qui les traversent en un point pour en déduire la quantité d'énergie au point considéré (Figure 5.a). De manière équivalente, le logiciel SPEOS permet de tracer des profils d'intensité lumineuse reçue par la grille en éclairage direct et indirect (voir exemple à la figure 5.b).

Le post-traitement consiste simplement à projeter la carte d'éclairement brute sur la surface réelle de la grille en métal déployé. Cette opération s'effectue simplement en multipliant la carte d'éclairement brute par une fonction $\Gamma$ (x,y) nulle partout sauf où le métal déployé se trouve, auquel cas elle est égale à l'unité. Nous obtenons les cartes d'intensité lumineuse à introduire dans notre modèle de purification (Figure 6).

Pour introduire ces cartes d'intensité, nous avons deux solutions :

1) introduire toutes les donnés dans un fichier qui sera utilisé dans le code de calcul ;
2) définir une fonction mathématique qui décrit la distribution spatiale de l'intensité lumineuse.

Nous optons pour la deuxième solution car elle permet, dans certaines conditions, d'obtenir des solutions analytiques. La fonction mathématique possède la forme suivante :

$$F_{SPEOS}(x,y) = \Gamma(x,y)\Re\left[\int g(k_x,k_y)\exp\left[i(k_x x + k_y y)\right]dk_x dk_y\right] \quad (1.14)$$

où :

- $F_{SPEOS}$ est la fonction spatiale décrivant la distribution d'intensité lumineuse sur la surface considérée ;
- x, y sont les coordonnées du point considéré sur la surface éclairée ;
- $g(k_x,k_y)$ est la transformée de Fourier de la fonction $F_{SPEOS}$ ;
- $k_x$, $k_y$ sont respectivement les nombres d'onde selon x, y ($2\pi/\lambda_x$, $2\pi/\lambda_y$) ;
- $\Re$ est la partie réelle de l'intégrale ;
- $\Gamma(x,y)$ est la fonction de projection de la carte d'éclairement brute sur la surface réelle du métal déployé.

Il s'agit d'une intégrale de Fourier. C'est cette fonction $F_{SPEOS}(x,y)$ que nous utilisons dans notre modèle de purification. Pour une distribution quelconque de l'intensité lumineuse, nous devons intégrer les ondes planes d'amplitude $g(k_x, k_y)$ sur tous les nombres d'onde (0, $\infty$). Dans notre étude, cette intégrale de Fourier peut être simplifiée en une série de Fourier car les caractéristiques géométriques du système étudié permettent de définir une périodicité.

En première approximation, nous considérons que l'intensité lumineuse est constante horizontalement, ce qui enlève la dépendance selon x dans l'intégrale de Fourier. De plus, la périodicité des lampes selon y permet de remplacer l'intégrale par une somme sur les longueurs d'onde multiples de la distance entre deux lampes.

$$F_{SPEOS}(x,y) = \Gamma(x, y)\sum A_j \cos(j\,\frac{2\pi}{d_{lampe}}\,y) \quad (1.15)$$

où $A_j$ représente l'amplitude de l'onde j, $d_{lampe}$ représente la distance entre deux rangées de lampes.

De manière générale, cette somme porte sur un nombre infini de contributions. Dans certains cas, seules les contributions des quelques premiers modes sont significatives ce qui permet de réduire la somme infinie à une somme sur un nombre fini de modes.

Pour obtenir la fonction $F_{SPEOS}$, nous faisons donc une analyse de Fourier sur les cartes d'éclairement brutes fournies par des simulations avec le logiciel SPEOS. Cette analyse nous donne les seuls paramètres non définis dans l'expression (1.15), à savoir les coefficients $A_j$ de la décomposition de Fourier.

7.3 Equation d'évolution et conditions limites

L'objectif de l'appareil de purification est de purifier en continu le volume d'air d'un espace considéré. Nous devons donc étudier le rendement de l'appareil de purification en régime stationnaire. Les équations d'évolution de la concentration d'acétaldéhyde sont, dès lors, des équations d'évolution stationnaire (équation d'advection-diffusion) :

$$\nabla.(\vec{u}C_A)=\nabla._{\llcorner}\ D_A(T,p^{tot},\mu^t)\nabla C_{A\lrcorner} \qquad (1.16)$$

où $\vec{u}$ (m/s) est le champ de vitesse de l'air, $C_A$(kg/m$^3$) la concentration du polluant A, $D_A$ le coefficient de diffusion du polluant A, constitué d'une contribution moléculaire et d'une contribution turbulente, avec $T(K)$ la température de l'air, $p^{tot}$(atm) la pression totale et $\mu^t$ la viscosité turbulente.

Sur les parties internes de l'encadrement extérieur de la paroi purificatrice, l'annulation du flux de masse est conservé comme condition limite. Sur les grilles en métal déployé, nous introduisons notre modèle de purification relatif à l'acétaldéhyde. L'introduction des données provenant du logiciel SPEOS ainsi que les fonctions d'interpolation des paramètres de purification permettent d'écrire l'expression de la décroissance temporelle de l'acétaldéhyde.

Pour l'étude du dimensionnement de la paroi purificatrice, nous devons imposer une concentration à l'entrée de la paroi. Désirant calculer le rendement de l'appareil de purification en régime stationnaire, nous imposons une concentration constante égale à 1 ppm$_{volumique}$. A la sortie, nous n'imposons aucune condition.

7.4 Définition du rendement de l'appareil de purification

Pour le dimensionnement de l'appareil de purification, nous devons définir un critère permettant d'évaluer les performances de purification. Pour ce faire, nous définissons le débit de polluant entrant et sortant de l'appareil :

$$Q_{A,entrée-sortie} = \iint\limits_{S_{entrée}-S_{sortie}} C_A\vec{u}.\vec{n}dS \quad (1.17)$$

où :

- $Q_{A,entrée-sortie}$ est le débit du polluant à l'entrée ou à la sortie de l'appareil de purification ;
- $C_A$ est la concentration du polluant à l'entrée ou à la sortie de l'appareil de purification ;
- $\vec{u}$ est le champ de vitesse à l'entrée ou à la sortie de l'appareil de purification ;
- $\vec{n}$ est la normale à la surface considérée pointant vers l'intérieur de l'appareil de purification à l'entrée et vers l'extérieur à la sortie.

A partir du débit de polluant, nous définissons le rendement de la paroi purificatrice pour le polluant A ($\eta_{A,paroi}$) :

$$\eta_{A,paroi} = \frac{Q_{A,sortie} - Q_{A,entrée}}{Q_{A,entrée}} \qquad (1.18)$$

Cette définition (1.18) nous permet d'avoir une grandeur variant entre 0, lorsque le système est totalement inefficace et 1 lorsque le système est parfaitement efficace, ce qui correspond à un appareil de purification ne rejettant aucun polluant dans l'espace à dépolluer.

La figure 7 résume l'algorithme d'optimisation de la paroi purificatrice.

7.5 Approche bi-dimensionnelle

La géométrie de la paroi purificatrice permet d'aborder le problème avec une approche bi-dimensionnelle au lieu d'une approche tri-dimensionnelle complète. La composante de vitesse selon l'axe $\vec{e}_y$ (Figure 1) est négligeable par rapport aux vitesses verticale et horizontale selon l'axe $\vec{e}_x$. L'étude de la paroi purificatrice s'effectue selon une coupe verticale. Les effets tri-dimensionnels sont négligeables sur toute la largeur de la paroi sauf aux deux extrémités où l'influence des parois latérales verticales se fait ressentir. Dans une première étude, nous négligeons ces effets 3D pour considérer un écoulement bi-dimensionnel dans toute la paroi.

Cette approche bi-dimensionnelle nous permet d'utiliser un code de calcul développé pour le cas 2D.

Dans la description de la paroi, nous avons défini la taille des mailles du métal déployé sur lequel le catalyseur, $TiO_2$, est déposé. Les grilles de métal déployé sont placées dans le plan O, $\vec{e}_y$, $\vec{e}_z$ qui est le plan perpendiculaire à la coupe verticale définie pour avoir une approche bi-dimensionnelle de l'écoulement. Pour modéliser le métal déployé, nous devons nous référer à son influence principale. Le métal déployé est utilisé pour déposer le dioxyde de titane. La caractéristique principale que nous devons conserver dans le modèle est le rapport ($S_{acier}$) entre la surface du métal déployé et la surface totale de la grille.

Nous modélisons le métal déployé par un mur perforé dont la dimension des interstices sont tels que le rapport de surface est conservé (Figure 8 : 1 grille et 3 lampes). Pour une paroi active composée de $n_{grilles}$ grilles de métal déployé, nous obtenons une surface totale de dioxyde de titane égale à :

$$S_{TiO2} = 2\, n_{grille}\, S_{acier}\, H_{paroi}\, L_{paroi} \qquad (1.19)$$

Le facteur 2 exprime le fait que le dioxyde de titane est déposé sur les deux faces d'une grille.

La figure 9 synthétise les courbes de rendement de la paroi purificatrice en fonction du nombre de rangées de lampes UVA, selon que l'on utilise une ou deux grilles et en ventilation naturelle ou forcée. Le rendement le plus élevé s'obtient avec le plus grand nombre de rangées de lampes (4 lampes), deux grilles et la ventilation naturelle.

L'utilisation d'un flux UVA comme activateur du film de TiO2 provoque une élévation de température à l'intérieur de la paroi purificatrice. Afin de déterminer les profils d'augmentation de température sur les parements extérieurs, on a utilisé un logiciel CFD permettant de calculer les champs d'écoulements et de températures, en tenant compte des flux de chaleur radiative, convective et de conduction. La figure 10 donne des exemples d'élévation de température $\Delta T = T - T0$ en fonction de la hauteur y de la paroi, selon le nombre de lampes et selon une convexion naturelle ou forcée.

## Revendications

1. Appareil de purification photocatalytique en continu de l'air d'une pièce d'habitation, se présentant de préférence sous forme d'une paroi murale (1), comprenant

   - une structure externe métallique, de préférence en acier ;
   - une ouverture (11) pour l'entrée de l'air à traiter située dans la partie inférieure de la face avant (10) de la paroi ;
   - une ossature métallique interne (4) sur laquelle est fixée une pluralité de lampes UVA (3) ;
   - un filtre (2) comprenant un support recouvert d'un film comprenant du dioxyde de titane ($TiO_2$) photocatalytique ;
   - une ouverture (12) pour la sortie de l'air purifié située dans la partie supérieure de la face avant (10) de la paroi, l'écoulement de l'air dans l'appareil étant assurée par circulation naturelle ou forcée par au moins un ventilateur (5) ;

   **caractérisé en ce que** ledit appareil comprend au moins une grille (2) en métal déployé recouverte d'un film comprenant du dioxyde de titane ($TiO_2$) majoritairement en phase anatase, pour maximiser la surface de photocatalyseur éclairée par la lumière UVA.

2. Appareil selon la revendication 1, **caractérisé en ce que** la structure externe métallique est réalisée dans un acier, ou possède une surface interne recouverte d'une couche mince, présentant un facteur de réflexion supérieur à 90% pour les longueurs d'onde inférieures à 400 nm.

3. Appareil selon la revendication 2, **caractérisé en ce que** la structure externe métallique est réalisée en acier inoxydable recuit brillant.

4. Appareil selon la revendication 1, 2 ou 3, **caractérisé en ce que** la face avant (10) de la structure externe a une largeur d'au moins 1,5 mètre, de préférence 2 mètres et **en ce que** les ouvertures d'entrée et de sortie d'air (11,12) ont la forme de fentes de largeurs égales et de valeur légèrement inférieure à celle de la largeur de ladite face avant (10) et de hauteurs supérieures à 3 cm, de préférence égales à 5 cm.

5. Appareil selon la revendication 4, **caractérisé en ce que** lesdites ouvertures (11,12) sont situées à moins de 10 cm, de préférence 5 cm, des extrémités respectivement basse et haute de ladite face avant (10).

6. Appareil selon l'un quelconque des revendications précédentes, **caractérisé en ce que** le métal déployé est un acier déployé et **en ce que** toute la surface des mailles (6) de cet acier déployé ($S_{acier}$) est recouverte du film de $TiO_2$, à l'exclusion de la surface de l'épaisseur de la maille, c'est-à-dire :

$$S_{acier} = [av_{maille} \sqrt{(\frac{DL_{maille}}{2})^2 + (\frac{DC_{maille}}{2})^2} - \frac{- av^2_{maille}}{2 \sin(2\,arctg(\frac{DC_{maille}}{DL_{maille}}))}],$$

où $DL_{maille}$, $DC_{maille}$ et $av_{maille}$ sont respectivement la diagonale longue, la diagonale courte et la lanière de la maille.

**7.** Appareil selon la revendication 6, **caractérisé en ce que** la maille (6) est choisie pour minimiser le rapport ($S_{acier}$) entre sa surface physique ($S_{acier}$) et sa surface totale ($S_{maille}$) :

$$S_{acier} = \frac{S_{acier}}{S_{maille}} = \frac{4}{DL_{maille}DC_{maille}} S_{acier},$$

ledit rapport valant de préférence 1/3.

**8.** Appareil selon l'une quelconque des revendications précédentes, **caractérisé en ce que** la grille (2) est maintenue verticalement avec des fixation situées uniquement sur le pourtour de la grille.

**9.** Appareil selon l'une quelconque des revendications précédentes, **caractérisé en ce que** les lampes UVA (3) sont disposées par séries de trois sur la largeur de la paroi.

**10.** Appareil selon l'une quelconque des revendications précédentes, **caractérisé en ce que** le ventilateur (5) est de type tangentiel à 90° et est situé en haut de la paroi, le nombre de ventilateurs étant choisi pour obtenir un renouvellement d'air d'au moins 30 m$^3$/heure/personne dans la pièce d'habitation.

**11.** Appareil selon la revendication 1, 2 ou 3, **caractérisé en ce qu'**il a la forme d'un cylindre de section circulaire, rectangulaire ou carrée présentant au moins un tube d'éclairage UVA selon l'axe du cylindre et entouré d'une grille en métal déployé recouverte de $TiO_2$ photocatalytique, la surface interne du cylindre ayant un facteur de réflexion supérieur à 90%.

**12.** Utilisation de l'appareil de purification selon l'une quelconque des revendications 1 à 11, pour la destruction par photocatalyse du dioxyde de titane de composés volatiles organiques tels que alcanes, alcools, aldéhydes, cétones, aromatiques et terpènes.

**13.** Utilisation de l'appareil de purification d'air selon l'une quelconque des revendications 1 à 10, dans le secteur du bâtiment, sous la forme d'un élément de structure ou décoratif tel qu'une paroi murale, une cloison, un plancher, un plafond ou un faux-plafond, ledit élément présentant une face externe métallique recouverte d'une finition telle que plâtre, peinture, papier peint.

**14.** Utilisation de l'appareil de purification d'air selon la revendication 11, sous la forme d'un conduit d'air.

**15.** Procédé d'optimisation du dimensionnement d'un élément purificateur d'air selon l'une quelconque des revendications 1 à 10, **caractérisé par** les étapes suivantes :

a)définition de la géométrie externe de l'appareil ;
b)définition du nombre de grilles (2) en acier déployé et du dispositif d'éclairage (3) ;
c)calcul de l'éclairement des grilles ; si l'intensité lumineuse n'est pas supérieure au seuil de tolérance fixé, retour à l'étape b) ;
d)calcul de l'écoulement de l'air et de la distribution de température ; s'il y a échauffement des parois en acier (11,15), retour à l'étape b) ;
e)calcul de l'évolution de la concentration des polluants ; si le rendement global de purification n'est pas supérieur à la limite prédéfinie, retour à l'étape b) ;
f)obtention de l'élément de purification ou dépollution de dimensions optimales.

Air

Air + polluants

$e_z$

$e_x$    $e_y$

FIG.1

FIG.2

FIG.3

FIG.4

FIG.5a

FIG.6

FIG.5b

Définition de la géométrie externe

↓

Définition du nombre de grille en acier
déployé et du système d'éclairage

↓

Calcul de l'éclairement des grilles

non

Intensité lumineuse
supérieure au seuil de
tolérance

Calcul de l'écoulement et de la distribution de
température

oui

Echauffement des parois en
acier

ok

Calcul de l'évolution des polluants

ok

Rendement global supérieur
à la limite définie

non

PAROI OPTIMALE

oui

FIG.7

12
sortie d'air

3

$DC_{maille}$  $s_{acier} DC_{maille}$

$sp_{maille}$

entrée d'air
11

FIG.8

FIG.9

Rendement (%)

Nombre de rangées de lampes

◆ 1 grille (naturel)  ■ 2 grilles (naturel)  ▲ 1 grille (forcé)  ✳ 2 grilles (forcé)

FIG.10

**Office européen**
**des brevets**

**RAPPORT DE RECHERCHE EUROPEENNE**

Numéro de la demande

EP 03 44 7240

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 0 590 477 A (TAKENAKA CORP) 6 avril 1994 (1994-04-06) * figure 14 * --- | 1-15 | B01D53/88 B01D53/86 A61L9/20 E04C2/00 |
| A | US 6 048 499 A (HIRAYAMA SHOJI) 11 avril 2000 (2000-04-11) * figure 3 * --- | 1-15 | |
| A | PATENT ABSTRACTS OF JAPAN vol. 2000, no. 12, 3 janvier 2001 (2001-01-03) & JP 2000 257185 A (NISSHIN STEEL CO LTD), 19 septembre 2000 (2000-09-19) * abrégé * ----- | 1-15 | |

**DOMAINES TECHNIQUES RECHERCHES (Int.Cl.7)**

B01D
A61L
E04C

Le présent rapport a été établi pour toutes les revendications

| Lieu de la recherche | Date d'achèvement de la recherche | Examinateur |
|---|---|---|
| LA HAYE | 30 mars 2004 | Faria, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................
& : membre de la même famille, document correspondant

EPO FORM 1503 03.82 (P04C02)

**EP 1 520 615 A1**

**ANNEXE AU RAPPORT DE RECHERCHE EUROPEENNE**
**RELATIF A LA DEMANDE DE BREVET EUROPEEN NO.**  EP 03 44 7240

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

30-03-2004

| Document brevet cité au rapport de recherche | | Date de publication | Membre(s) de la famille de brevet(s) | | | Date de publication |
|---|---|---|---|---|---|---|
| EP 0590477 | A | 06-04-1994 | CA | 2106510 | A1 | 23-03-1994 |
| | | | DE | 69311866 | D1 | 07-08-1997 |
| | | | DE | 69311866 | T2 | 05-02-1998 |
| | | | EP | 0590477 | A1 | 06-04-1994 |
| | | | JP | 6278241 | A | 04-10-1994 |
| | | | US | 5595813 | A | 21-01-1997 |
| | | | US | 5643436 | A | 01-07-1997 |
| | | | JP | 3316048 | B2 | 19-08-2002 |
| | | | JP | 6198196 | A | 19-07-1994 |
| US 6048499 | A | 11-04-2000 | AUCUN | | | |
| JP 2000257185 | A | 19-09-2000 | AUCUN | | | |

Pour tout renseignement concernant cette annexe : voir Journal Officiel de l'Office européen des brevets, No.12/82

26

EPO FORM P0460